(19) Europäisches Patentamt
European Patent Office
Office européen des brevets

(11) Veröffentlichungsnummer : **0 474 712 B1**

(12) **EUROPÄISCHE PATENTSCHRIFT**

(45) Veröffentlichungstag der Patentschrift :
**22.09.93 Patentblatt 93/38**

(51) Int. Cl.$^5$ : **A61K 31/685**

(21) Anmeldenummer : **90908504.5**

(22) Anmeldetag : **31.05.90**

(86) Internationale Anmeldenummer :
**PCT/EP90/00870**

(87) Internationale Veröffentlichungsnummer :
**WO 90/14829 13.12.90 Gazette 90/28**

# (54) LYSOLECITHINDERIVATE ZUR BEHANDLUNG VON AUTOIMMUNERKRANKUNGEN.

(30) Priorität : **02.06.89 DE 3918082**

(43) Veröffentlichungstag der Anmeldung :
**18.03.92 Patentblatt 92/12**

(45) Bekanntmachung des Hinweises auf die Patenterteilung :
**22.09.93 Patentblatt 93/38**

(84) Benannte Vertragsstaaten :
**AT BE CH DE DK ES FR GB IT LI LU NL SE**

(56) Entgegenhaltungen :
**WO-A-87/01257**
**DE-A- 2 009 342**
**DE-A- 2 009 343**
**DE-A- 2 619 686**
**Lipids, vol. 22, no. 11, 1987, R. Andreesen et al., pp. 836-841**
**The J: Biol. Chemistry, vol. 261, no. 17, 18 June 1986, The Am. Soc. of Biol. Chemists, Inc. (US) D.B.J. Herrmann et al., pp. 7742-7747**
**Lipids, vol. 22, no. 11, 1987, I. Kudo et al., pp. 862-867**
**Lipids, vol. 22, no. 11, 1987, M.R. Berger et al., pp. 935-942**

(56) Entgegenhaltungen :
**The Merck Manual of Diagnosis and Therapy, 15th ed. 1987, Merck Sharp & Dohme Research Laboratories, Division of Merck & Co., Inc. (Rahway, N.J., US) pp. 1239-12, chapter 108**
**Allgemeine und Spezielle Pharmakologie und Toxikologie, 5th ed. 1987, Bi Wissenschaftsverlag (Mannheim, DE) Forth, Henschler & Rummel, pp. 737-738**

(73) Patentinhaber : **Max-Planck-Gesellschaft zur Förderung der Wissenschaften e.V.**
**Bunsenstrasse 10**
**D-37073 Göttingen (DE)**

(72) Erfinder : **MODOLELL, Manuel**
**Rosbaumweg 50**
**D-7800 Freiburg (DE)**
Erfinder : **MUNDER, Gerhard**
**Bergstrasse 38 a**
**D-7803 Gundelfingen (DE)**

(74) Vertreter : **Huber, Bernhard, Dipl.-Chem. et al**
**Patentanwälte H. Weickmann, Dr. K. Fincke F.A. Weickmann, B. Huber Dr. H. Liska, Dr. J. Prechtel Kopernikusstrasse 9 Postfach 86 08 20**
**D-81635 München (DE)**

EP 0 474 712 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft die Verwendung von Derivaten des Lysolecithins zur Herstellung einer pharmazeutischen Zubereitung zur Behandlung von rheumatoider Arthritis oder ankylosierender Spondylitis.

Aus der DE-OS 20 09 342 und der DE-OS 20 09 343 ist es bereits bekannt, daß synthetische Lysolecithin-Verbindungen als immunologische Adjuvantien und zur Steigerung der Resistenz verwendet werden können. Desweiteren ist es aus der DE-OS 26 19 686 bekannt, daß derartige Al-kyllysophospholipide als Antitumormittel wirksam sind. Zudem ist es bekannt, daß es nach Applikation von Lysophosphatiden zur Bildung von aktivierten Zellen kommt, die die Resistenz des Körpers gegen unerwünschte Einflüsse steigern können. Schließlich ist in der DE-OS 35 30 767 die Verwendung von synthetischen Lysolecithinverbindungen zur Behandlung der Multiplen Sklerose beschrieben.

Bei allen immunologischen Reaktionen des Organismus spielen T-Lymphozyten eine zentrale Rolle. Dabei kann ihre Aufgabe und ihre Wirkungsweise in zwei wesentliche Wirkmechanismen unterteilt werden, nämlich

1. die direkte Abwehr eines Fremdkörpers wie Bakterien, Viren, Parasiten und anderen, ein Antigen aufweisenden Substanzen, wobei die T Lymphozyten direkt durch Effektormechanismen beteiligt sind und
2. die Regulierung der Immunantwort, damit diese nicht überschießt.

In beiden Fällen ist es für die Wirkungsweise der T-Lymphozyten charakteristisch, daß diese eine hohe Spezifität aufweisen, d.h. daß diese Zellen die Eigenschaft haben, ein einziges, ganz bestimmtes Antigen zu erkennen. Bevor die T-Zellen jedoch diese Eigenschaft zeigen, ist es notwendig, daß sie zuerst aus einem ruhenden Zustand heraus aktiviert werden, was eine Proliferation und eine Differenzierung voraussetzt.

T-Lymphozyten können nun anhand definierter Membranstrukturen, die durch Proteine gebildet werden, in zwei große Untergruppen aufgeteilt werden, nämlich den $CD4^+$ und den $CD8^+$ T-Zellen. Die $CD4^+$ werden als Helfer bzw. als Inducer-T-Zellen bezeichnet und sind unter anderem verantwortlich für die Erkennung von Fremdproteinen und die Auslösung von Abwehrreaktionen gegen Antigene. Die $CD8^+$-Zellen, die als Suppressor- bzw. als cytotoxische T-Zellen bezeichnet werden, sind u.a. für die Erkennung und die Zerstörung von virusinfizierten Zellen verantwortlich.

Es hat sich nun gezeigt, daß insbesondere bei höherentwickelten Arten des Tierreiches T-Zellen auftreten, die autoreaktive Eigenschaften aufweisen. Dies bedeutet, daß solche T-Lymphozyten gegen körpereigene Strukturen des Organismus gerichtet sind, und somit darauf vorprogrammiert sind, körpereigene Stoffe und Zellen anzugreifen. Normalerweise bleiben diese autoreaktiven T-Zellen durch regulierende Einwirkungen von anderen Zellen oder Faktoren inaktiv, so daß ein Gleichgewicht zum Schutz des Organismus entsteht. Wird dieses Gleichgewicht jedoch gestört, so können dadurch Autoimmunerkrankungen induziert werden. Dies führt zur Bildung von autoaggressiven Zellen oder Autoantikörpern, die schließlich die Autoimmunkrankheit auslösen. Es ist jedoch bisher nicht bekannt, welche Antigene beim Menschen für die Auslösung dieser Symptome verantwortlich sind.

Solche Autoimmunerkrankungen sind bislang mit immunsuppressiven Substanzen wie Corticostereoiden, Cyclophosphamiden, Cyclosporin A und ähnlichen behandelt worden. Es hat sich doch gezeigt, daß diese Substanzen nicht nur das gesamte Immunsystem inhibieren, sondern zusätzlich auch erhebliche Nebenwirkungen, wie eine Suppression des Knochenmarks, Carcinogenität sowie eine hohe Toxizität aufweisen.

Der Erfindung liegt nun die Aufgabe zugrunde, ein weiteres Mittel zur Behandlung von Autoimmunkrankheiten bereitzustellen, welches die zuvor genannten Nachteile nicht aufweist.

R. Andreesen und P. Munder (New Trends Lipid Mediators Res. Basel, Karger, 1988, Vol. 1, Seiten 16 bis 29 und Immunbiol. (1979) 156, 498- 508) konnten bereits nachweisen, daß Alkyllysophospholipide in der Lage sind, die unspezifische Proliferation von Lymphozyten zu inhibieren.

Überraschenderweise wurde nun gefunden, daß die Proliferation gerade von solchen spezifischen autoreaktiven T-Zellen, die für die Autoimmunreaktionen verantwortlich sind, durch ganz bestimmte Alkyllysophospholipide gehemmt werden kann.

Gegenstand der Erfindung ist somit die Verwendung von Verbindungen der allgemeinen Formel I

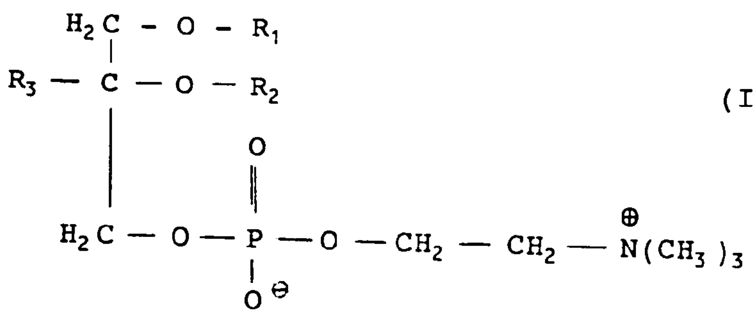

(I)

in der
$R_1$ Alkyl mit 12 bis 18 Kohlenstoffatomen,
$R_2$ Alkyl mit 1 bis 8 Kohlenstoffatomen und
$R_3$ H oder Alkyl mit 1 bis 3 Kohlenstoffatomen darstellt, zur Herstellung einer pharmazeutischen Zubereitung zur Behandlung von rheumatoider Arthritis oder ankylosierender Spondylitis.

Die Alkylgruppen $R_1$, $R_2$ und/oder $R_3$ können verzweigt sein und sind vorzugsweise jedoch geradkettig. In einer Verbindung der allgemeinen Formel I ist $R_2$ vorzugsweise ein Alkylrest mit 1 bis 3 Kohlenstoffatomen und insbesondere Methyl und/oder $R_3$, vorzugsweise H. Bevorzugte Verbindungen sind insbesondere die Verbindung der Formel I mit $R_1$ = n-Hexadecyl, und $R_2$ = Methyl und $R_3$ = H (Verbindung II), und in erster Linie die Verbindung der Formel I mit $R_1$ = n-Octadecyl, und $R_2$ = Methyl und $R_3$ = H (Verbindung III).

Die Herstellung der Verbindungen der Formel I ist bekannt und kann auf einem der in der Literatur beschriebenen Wege erfolgen; vgl. z.B. D. Arnold et al., Liebigs Ann. Chem. 709, 234-239 (1967); H.U. Weltzien und O. Westphal, Liebigs Ann. Chem. 709, 240-243 (1967).

Es hat sich gezeigt, daß die erfindungsgemäß hergestellte pharmazeutische Zubereitung sich besonders gut zur Behandlung der rheumatoiden Arthritis oder der ankylosierenden Spondylitis eignet.

Bei dieser sehr häufig auftretenden Erkrankung handelt es sich um eine progrediente chronische Polyarthritis einer Allgemeinerkrankung, die schleichend oder in Schüben verläuft. Sie tritt meist im 3. bis 5. Lebensjahrzehnt auf, wobei Frauen etwa dreimal so häufig als Männer betroffen sind. Diese Erkrankung kann zu schwersten Verkrüppelungen führen.

Wie bereits oben diskutiert, ist ET-18-$OCH_3$ insbesondere zur Behandlung von rheumatoider Arthritis und ankylosierender Spondylitis geeignet. Die Wirkung von ET-18-$OCH_3$ wurde bei der Proteoglykan-induzierten progressiven Polyarthritis von Balb/c-Mäusen untersucht. Dieses Tiermodell weist eine starke Übereinstimmung zur rheumatoiden Arthritis und ankylosierenden Spondylitis bei Menschen auf, wie durch klinische Untersuchungen, z.B. radiographische Analyse und szintigraphische Knochenuntersuchungen und durch histopathologische Studien von Gelenkverbindungen und der Wirbelsäule nachgewiesen wurde (Glant et al., Arthritis rheum. 30 (1987), 201-212; Mikecz et al., Arthritis rheum. 30 (1987), 306-318). Die Arthritis beginnt als polyartikulare Synovitis bei bilateralen, kleinen peripheren Gelenken und beinhaltet eine Spondylitis, die mit extensiver Zerstörung von Knorpel und Knochen innerhalb des Gelenks progressiv wird. Die anfänglichen äußeren Symptome von Gelenkentzündung (Schwellung und Rötung) erscheinen nach der dritten oder vierten intraperitonealen Injektion von arthritogenen Proteoglykanen.

Das Auftreten von Arthritis in Balb/c-Mäusen ist vom Auftreten sowohl von zellulärer als auch humoraler Immunantwort gegenüber Knorpelproteoglykanen der Wirtsmaus abhängig. Zirkulierende Autoantikörper treten jedoch nur bei hyperimmunisierten arthritischen Tieren auf, wenn der Knorpelabbau mehr oder weniger zu Tage tritt. Diese Autoantikörper reagieren mit intakten (nicht abgebauten) und Chondroitinase ABC behandelten Knorpel-Proteoglykanen der Maus und zeigen auch eine Kreuzreaktion mit dem immunisierenden Proteoglykan (Mikecz et al., Arthritis rheum, 30 (1987), 306-318).

Eine Vorbehandlung mit ET-18-$OCH_3$ schützt die Tiere vor der Krankheit oder, sofern die Behandlung während der Immunisierung mit arthritogenem Proteoglykan begonnen wurde, wird die Entwicklung von Arthritis erheblich verzögert. Weiterhin waren die klinischen Symptome, wie z.B. Rötung und Schwellungen, weniger intensiv, sofern sich Arthritis bei der behandelten Gruppe entwickelte. Es waren auch weniger periphere Gelenke als bei nicht behandelten Tieren betroffen. Der Beginn der Behandlung mit ET-18-$OCH_3$ verringerte den Spiegel an zirkulierenden (Auto)-Antikörpern und unterdrückte die Produktion spezifischer Antikörper während der gesamten Experimentdauer. T-Lymphozyten aus Tieren, die zuvor mit ET-18-$OCH_3$ behandelt wurden, proliferierten nicht in Gegenwart von für die Immunisierung verwendetem Antigen. Die unspezifische T-Zell-Stimulierung mit Concavalin A wurde ebenfalls signifikant unterdrückt.

Es wurde auch gefunden, daß sich mit Hilfe der erfindungsgemäß zu verwendenden

Alkyllysophospholipide autoreaktive T-Zellen inhibieren lassen und zwar gleichgültig, ob es sich dabei um $CD4^+$-oder $CD8^+$-Zellen handelt, wobei diese Inhibition durch eine Störung des Phosphatidylcholin-Stoffwechsels der Zelle hervorgerufen wird. Insbesondere ist bemerkenswert, daß diese Störung nur für aktivierte T-Zellen spezifisch ist und dabei nicht aktivierte T-Zellen in ihrer Syntheseleistung nicht beeinflußt werden. Da die Proliferation von autoreaktiven T-Zellen eine unbedingte Voraussetzung dafür ist, daß diese autoaggressiv wirken, läßt sich somit mittels der durch Alkyllysophospholipid-induzierten Hemmung eine Immunsuppression durchführen.

Die Erfindung wird durch die folgenden Beispiele in Verbindung mit der Zeichnung näher erläutert. In der Zeichnung stellen dar:

Fig. 1 eine graphische Darstellung der Hemmung der Proliferation von autoreaktiven $CD4^+$-Zellen durch verschiedene Wirkstoffe in Abhängigkeit von deren Menge;

Fig. 2 eine Darstellung analog Fig. 1 in Abhängigkeit von der Versuchsdauer;

Fig. 3 eine Darstellung analog Fig. 1 für die Proliferationshemmung von CTLL1-Zellen;

Fig. 4 eine graphische Darstellung der de-novo-Syntheseleistung von T-Lymphozyten (Cholineinbau) in Gegenwart von ET-18-$OCH_3$ in Abhängigkeit vom Aktivierungszustand der Zellen;

Fig. 5 eine graphische Darstellung des Abbaus von Phosphatidylcholin durch AR17 T-Zellen in Gegenwart von ET-18-$OCH_3$;

Fig. 6 eine Darstellung analog Fig. 5 für nicht aktivierte T-Zellen;

Fig. 7 eine graphische Darstellung eines Lymphozyten-Stimulierungstests von immunisierten Balb/c-Mäusen der Gruppen 2 bis 5;

Fig. 8 eine graphische Darstellung des Antikörperspiegels in Tieren der Gruppe 4, die mit Proteoglykanen von bovinen Gelenkknorpeln (BAC-PG) immunisiert wurden;

Fig. 9 eine Darstellung der Wirkung von ET-18-$OCH_3$ auf den Antikörperspiegel im Serum von Balb/c-Mäusen der Gruppe 5, die mit Proteoglykanen von bovinen Gelenkknorpeln (BAC-PG) immunisiert wurden;

Fig. 10 eine graphische Darstellung des Antikörperspiegels im Serum von Tieren der Gruppe 7, die mit Proteoglykanen von menschlichen Osteophyten-Knorpeln (HYAC-PG) immunisiert wurden;

Fig. 11 eine Darstellung analog Fig. 7 von Mäusen der Gruppen 6 bis 9;

Fig. 12 eine graphische Darstellung der Wirkung von ET-18-$OCH_3$ auf den Antikörperspiegel im Serum von Balb/c-Mäusen der Gruppe 8, die mit HYAC-PG immunisiert wurden;

Fig. 13 eine graphische Darstellung des Antikörperspiegels im Serum von Balb/c-Mäusen, die mit ET-18-$OCH_3$ vorbehandelt wurden und anschließend mit HYAC-PG immunisiert wurden.

**Beispiel 1**

Die hierbei verwendeten autoreaktiven $CD4^+$ T-Lymphozyten sind für die Auslösung von experimenteller Autoimmuno-Arthritis (auch Adjuvansarthritis genannt) in der Ratte verantwortlich. Sie sind nach der Methode von J. Holoshitz et al., Science, Vol. 219, (1983), Seiten 56 bis 58, isoliert worden. Die Proliferation wurde mit dem Antigen PPD (Purified Protein Derivative, gereinigte Fraktion aus Tuberkelbakterien) induziert.

Hemmung der Proliferation von $CD4^+$-Zellen durch Alkyllysophospholipide während der spezifischen Reststimulation in Gegenwart eines Antigens:

$1x10^3$ T-Lymphozyten (AR 17) werden in RPMI 1640 + 10 % fötales Kälberserum + 1 µg Antigen während 48 Stunden kultiviert. Als Antigenpresenterzellen werden $1x10^6$ bestrahlte (30 Gy) Thymozyten kokultiviert. Nach dieser Zeit wird pro Kultur 0,5 µCi $^3$H-Thymidin zugefügt und nach weiteren 6 Stunden Kultivation wird der Einbau des Thymidins in die DNS der Zelle gemessen.

Das Ergebnis ist in Figur 1 dargestellt. Daraus ist ersichtlich, daß die auf diese Weise ausgelöste Proliferation der AR 17-Zellen durch die Substanz 1-Octadecyl-2-methyl-glycero-3-phosphocholin, im folgenden ET-18-$OCH_3$ genannt, gehemmt wird.

**Beispiel 2**

AR 17-Zellen, die wie in Beispiel 1 beschrieben, isoliert worden sind, werden durch Zugabe von Wachstumsfaktoren (IL-2) zur Proliferation in Kultur stimuliert. Dabei wird die Hemmung dieser Proliferation mittels Alkyllysophospholipiden untersucht. Der Versuch wurde wie folgt durchgeführt:

$1x10^3$ T-Lymphozyten (AR 17) werden in RPMI 1640 + 10 % fötales Kälberserum + 10 % Kulturüberstand aus Con A stimulierten Milzzellen (IL 2) während 24 bzw. 48 Stunden kultiviert. Nach dieser Zeit wird pro Kultur 0,5 µCi $^3$H-Thymidin zugefügt und nach weiteren 6 Stunden Kultivation wird der Einbau des Thymidins in die DNS der Zelle gemessen.

Das Ergebnis ist in Fig. 2 dargestellt. Daraus ist ersichtlich, daß die Substanz ET-18-$OCH_3$ auch in diesem Fall eine ausgeprägte Hemmwirkung bezüglich der Proliferation zeigt.

**Beispiel 3**

Es wurden cytotoxische T-Lymphozyten (CTLL1), wie von M.M. Simon et al., Eur. J. Immunol. (1986), 16, 1269-1276 beschrieben, isoliert und die Proliferationshemmung dieser Zellinie durch Alkyllysophospholipide untersucht. Bei der hierbei verwendeten Zellinie handelt es sich um autoreaktive $CD8^+$ T-Zellen. Zur Durchführung des Versuches wurden $1x10^3$ T-Lymphozyten (CTLL-1) in RPMI 1640 + 10 % fötales Kälberserum + 10 % Kulturüberstand aus Con A stimulierten Milzzellen (IL2) während 48 Stunden kultiviert. Nach dieser Zeit wird pro Kultur 0,5 µCi $^3$H-Thymidin zugefügt und nach weiteren 6 Stunden Kultivation wird der Einbau des Thymidins in die DNS der Zelle gemessen. Die Ergebnisse sind in Fig. 3 in Prozentwerten bezüglich der Kontrollmessungen dargestellt. Wie aus dieser Abbildung zu entnehmen ist, zeigt sowohl ET-18-OH, als auch ET-18-$OCH_3$ eine ausgeprägte Hemmung der Proliferation.

**Beispiel 4**

Es wurde der Einbau von radioaktiv markiertem Cholin in das Phosphatidylcholin untersucht und auf diese Weise die de-novo-Syntheseleistung der Zellen bestimmt. Dabei wurden $1x10^6$ T-Lymphozyten werden in cholinfreiem DMEM (Dulbecco's Modifikation von Eagle's Medium) + 10 % fötalem Kälberserum + 10 % Kulturüberstand aus Con A stimulierten Milzzellen (IL 2) + 0,51 Ci $^{14}$C-Cholin während des angegebenen Zeitraumes kultiviert. Die Phospholipide der Zellen werden extrahiert, mit Dünnschichtchromatografie getrennt und die Radioaktivität der Phosphatidylcholinbande wird mit einem DC-Scanner gemessen. Die Ergebnisse sind in Fig. 4 dargestellt. Dabei zeigen die gestrichelten Linien den Cholineinbau an unstimulierten T-Zellen und die durchgezogenen Linien den Einbau an stimulierten $CD4^+$ T-Zellen (AR 17) an. Die einzelnen Ergebnisse sind in Prozent, bezogen auf die Kontrollwerte, angegeben. Dabei zeigt es sich, daß überraschenderweise ET-18-$OCH_3$ nur die Synthese von aktivierten autoreaktiven AR 17 T-Zellen hemmt, wohingegen die nichtaktivierten T-Lymphozyten, die nicht an der Autoimmunreaktion beteiligt sind, von dieser Substanz in ihrer Syntheseleistung unbeeinflußt bleiben.

**Beispiel 5**

Die Fettsäure-Zusammensetzung des Phosphatidylcholins wird ständig durch die Einwirkung von Phospholipase A als deacylierendes- und der Acyltransferase als reacylierendes Enzym erneuert. Um diesen Stoffwechsel zu erfassen, wurden die T-Zellen im voraus mit C14 Ölsäure inkubiert, so daß das Phosphatidylcholin durch Einbau dieser Fettsäure radioaktiv markiert war.

Es wurde nun der Abbau von Phosphatidylcholin in AR 17 T-Zellen nach Zugabe von verschiedenen Alkyllysophospholipiden bestimmt. Dabei wurden $1x10^6$ T-Lymphozyten in DMEM + 1 % fötales Kälberserum + 0,1 µCi $^{14}$C-Ölsäure während 4 Stunden kultiviert. Danach werden die markierten Zellen mit verschiedenen Alkyllysophospholipiden während 24 bzw. 48 Stunden in DMEM + 10 % fötalem Kälberserum + 10 % Kulturüberstand aus Con A stimulierten Milzzellen (IL 2) weiter kultiviert. Die Phospholipide der Zellen werden extrahiert, mit Dünnschichtchromatografie getrennt und die Radioaktivität der Phosphatidylcholinbande wird mit einem DC-Scanner gemessen. Die Ergebnisse sind als Prozent, bezogen auf die Kontrollwerte, in Fig. 5 dargestellt. Daraus ist ersichtlich, daß die Zellen, die mit ET-18-$OCH_3$ inkubiert wurden, einen hohen Verlust an Radioaktivität aufweisen, was einen Verlust an Phosphatidylcholin bedeutet. Wie aus Fig. 6 zu entnehmen ist, zeigen im Gegensatz dazu nicht aktivierte T-Zellen keine Wirkung gegenüber einer Inkubation mit dieser Substanz.

**Beispiel 6**

Wirkung von ET-18-$OCH_3$ auf die Ausbildung von Proteoglykan-induzierter Arthritis

Induktion von primärer Arthritis:

Mit Chondroitinase ABC gespaltenes Proteoglykan von menschlichen Chondrophyten (entsprechend einem Proteoglykan aus unreifen menschlichen Gelenkknorpeln (HYAC)) wurde zur Immunisierung von 5 bis 6 Wochen alten weiblichen Balb/c-Mäusen (Charles River Colony, Montreal, Quebec) wie bereits beschrieben verwendet (Glant et al., Arthritis rheum. 30 (1987), 201-212; Mikecz et al., Arthritis rheum. 30 (1987), 306-318). Es wurden 100 µg Proteoglykan-Protein von Chondroitinase ABC gespaltenem HYAC in 100 µl phosphatgepufferter Salzlösung (PBS), pH 7,2 und mit Freund's komplettem Adjuvans in einer 1:1-Emulsion

mit PBS intraperitoneal injiziert. An den Tagen 9, 35 und 65 wurden sie erneut dreimal mit dem Antigen in Freund's inkomplettem Adjuvans injiziert. Weibliche Balb/c-Kontrollmäuse wurden mit nicht arthritogenen (bovinem Gelenk) Proteoglykan (BAC-TG) oder mit phosphatgepufferter Salzlösung (PBS) und Adjuvans ohne Proteoglykan-Antigene immunisiert.

Die Gruppen dieses Experiments sind in Tabelle 1 genannt. Ursprünglich wurden 10 Tiere in Gruppe 9 mit ET-18-$OCH_3$ vorbehandelt und anschließend mit arthritogenem Proteoglykan aus menschlichen Chondrophyten behandelt, das bei den Gruppen 6 (immunisiert, unbehandelt), 7 (immunisiert, unbehandelt, aber täglich mit Milch gefüttert) und 8 (immunisiert und mit in Milch gelöstem ET-18-$OCH_3$ behandelt) verwendet wurde.

T a b e l l e   I

Experimentelle Gruppen, Immunisierung und Behandlung von Balb/c-Mäusen

| Gruppe Nr. | Zahl der Tiere | Immunisierung mit | Immunstatus: Antikörper gegen | | klinischer Status | Behandlung mit ET-18-$OCH_3$ |
|---|---|---|---|---|---|---|
| | | | HYAC-PG | Maus-PG | | |
| 1 | 5 | | - | - | nicht arthritisch | - |
| 2 | 5 | | Salzlösung | - | nicht arthritisch | Lösungsmittel |
| 3 | 5 | | " | - | nicht arthritisch | + |
| 4 | 5 | BAC-PG | + | ± | nicht arthritisch | Lösungsmittel |
| 5 | 6 | BAC-PG | + | ± | nicht arthritisch | + |
| 6 | 5 | HYAC-PG | + | + | arthritisch | - |
| 7 | 6 | HYAC-PG | + | + | arthritisch | Lösungsmittel |
| 8 | 6 | HYAC-PG | + | + | spät und schwach arthritisch | + |
| 9 | 10 | HYAC-PG | + | + | nicht arthritisch | vorbehandelt |

Den Tieren wurde eine Emulsion von Salzlösung und Freund'schem Adjuvans injiziert oder sie wurden mit Proteoglycan-Antigenen in Freund's Adjuvans immunisiert.
Abkürzungen:
HYAC-PG = Proteoglycan von menschlichen Chondrophyten (Osteophyten-Gelenke)
BAC-PG = Proteoglycan von bovinen Gelenkknorpeln
Salzlösung = Phosphat-gepufferte Salzlösung, pH 7,4 (0,01 mol/l $PO_4$ in 0,14 mol/l NaCl)
Lösungsmittel = Milch (die ansonsten zur Lösung von ET-18-$OCH_3$ verwendet wurde).

Tiere aus zwei Gruppen (Nr. 4 und Nr. 5) wurden mit BAC-PG immunisiert und mit ET-18-$OCH_3$ behandelt oder nicht behandelt, während 15 Mäuse in drei weiteren Gruppen als negative Kontrolle dienten (Tabelle 1). In jeder Gruppe waren 5 bis 10 weibliche, zu Beginn des Experiments 5 bis 6 Wochen alte Balb/c-Mäuse. Die Tiere wurden mit ET-18-$OCH_3$, gelöst in Milch (fettarme Milch), 12 Wochen lang (5 bis 6 Wochen lang 25 mg/kg und 6 Wochen lang 20 mg/kg) durch eine Oesophagus-Sonde (orale Verabreichung des Arzneimittels) behandelt. Die Dosis von ET-18-$OCH_3$ wurde von 25 mg/kg auf 20 mg/kg verringert, da sich der physische Zustand der behandelten Balb/c-Mäuse von der zweiten Woche der Behandlung an ständig verschlechterte, und 4 Tiere der Gruppe 9 und zwei Tiere der Gruppe 8 in den Wochen 3 bis 4 der Behandlung mit ET-18-$OCH_3$ verlorengingen. Die Tiere (abgesehen von Gruppe 9, siehe oben) wurden von Woche 5 bis Woche 17 des Experiments mit ET-18-$OCH_3$ behandelt und dann getötet.

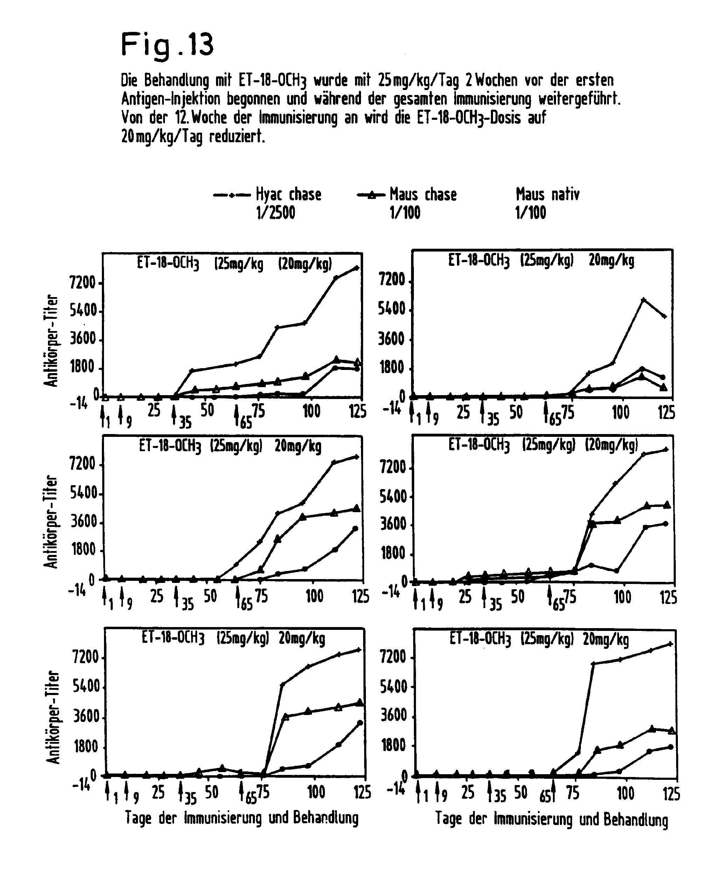

Jede Zeitangabe, die in diesem Bericht angegeben ist (siehe Figuren 8 bis 10, 12 und 13), beginnt am Tag der ersten Injektion.

Serumproben, Antikörpertests:

An den Tagen 1, 9, 19, 27, 35, 44, 55, 65, 77, 85, 97 und 112 wurden Serumproben aus dem retro-orbitalen, venösen Plexus gesammelt. Die Proben wurden bei -20°C bis zum endgültigen Ausbluten am Tag 122 aufbewahrt, wobei dann die Antikörpertiter in 13 Serumproben jedes Tiers gleichzeitig bestimmt und verglichen wurden.

Die Antikörper in den Seren wurden durch einen Radioimmuntest in Lösung bestimmt, wobei $^{125}$I-markierte Knorpel-Proteoglykane von menschlichen Neugeborenen (Chondroitinase ABC behandelt (=chase)) und von der Maus (intakt (=nativ) und Chrondroitinase ABC behandelt) verwendet wurden. Jedes Serum wurde bei einer Verdünnung von 1:100, 1:500 und 1:2500 und gelegentlich bei 1:12500 wie bereits beschrieben, getestet (Mikecz et al., supra, Glant et al., Biochem. J. 234, 31-41). Dazu wurden 100 µl von verdünntem Serum mit $^{125}$I markiertem Proteoglykan (10000 Ipm in 50 µl) 4 Stunden lang bei Raumtemperatur inkubiert. Nach der Inkubation wurden 25 µl Protein A-tragender Staphylococcus aureus (5 % Gewicht/Volumen) zugegeben, 30 Minuten lang bei 37°C inkubiert und zweimal gewaschen. Die Radioaktivität der Rückstände wurde in einem Backman Gammazähler bestimmt.

Tests auf Lymphozyten-Reaktivität:

Die Antwort von Splenozyten auf Concavalin A (ConA als Maus-T-Zellen-Mitogen) und Knorpel-Proteoglykan-Antigenen (BAC-PG und HYAC-PG) wurde wie bereits beschrieben bestimmt (Mikecz et al., supra). Die Stimulierung von Lymphozyten wurde als Index ausgedrückt, der ein Verhältnis der Ipm von antigen oder mitogen stimulierten Kulturen zu den Ipm von nicht stimulierten Kulturen ist.

Bewertung der Arthritis:

Die Gliedmaßen aller Mäuse (arthritisch und nicht arthritisch, immunisiert und nicht immunisiert) wurden täglich untersucht, um klinische arthritische Veränderungen nachzuweisen, die, wie bereits beschrieben, dokumentiert wurden (Glant et al., Arthritis Rheum. 30 (1987), 201-212). Schwellung und Rötung, als erste klinische Symptome des Auftretens von Arthritis, sowie die Dicke (Durchmesser) von Knien, Knöcheln, Handwurzel und die dorso-volare Dicke der Hand wurden aufgezeichnet.

Klinische und histologische Untersuchungen:

Die Zahl der roten und weißen Blutkörperchen (Erythrozyten und Leukozyten), sowie das Blutbild der weißen Blutkörperchen wurden vor dem Experiment, bei Woche 11 (nach 6 Wochen Behandlung) und am Ende des Experiments (am Tag 122) bestimmt.

Die Glieder, Schwänze, Lumbarspina und Organe wurden fixiert, die Knochen wurden dekalzifiziert und zur Sektion für histologische Studien vorbereitet.

Ergebnisse:

Gruppen 1, 2 und 3 (nicht immunisiert, nicht arthritische Kontrolltiere):

Diese Balb/c-Mäuse (jeweils 5 in einer Gruppe) wurden nicht mit Proteoglykan-Antigen immunisiert, aber den Tieren der Gruppen 2 und 3 wurde eine Emulsion von physiologischer Salzlösung und Freund'schem Adjuvans (Tabelle 1) injiziert. Keines dieser Tiere erzeugte Antikörper gegen irgendeine Knorpel-Komponente, einschließlich Proteoglykane und Collagen. Typ II, und sie entwickelten keine Arthritis.

Die behandelten Tiere in Gruppe 3 verloren Körpergewicht und wurden leicht anämisch (Tabelle 2) (vermutlich aufgrund der häufigen Blutentnahme), während sich keine anderen klinischen Symptome oder Laborparameter änderten. Nur die Lymphozyten-Stimulierung mit einem T-Zellen-Mitogen (ConA) wurde signifikant verringert (Fig. 7).

Tabelle II

Vergleich des Körpergewichts, der Erythrozytenzahl im peripheren Blut und der intermalleolaren Durchmesser von arthritischen und nicht arthritischen Balb/c-Mäusen an Tagen 1, 71 und 122 des Experiments

| Gruppen | Tag des Experiments | Körpergewicht (g) | Erythrozytenzahl ($x10^6/\mu l$) | Intermalleolarer Durchmesser (mm) |
|---|---|---|---|---|
| 2 | 1 | 19,9 ± 2,7 | 9,13 ± 0,62 | 3,16 ± 0,05 |
| | 71 | 27;2 ± 1,2 | 8,78 ± 0,49 | 3,14 ± 0,05 |
| | 122 | 27,4 ± 1,6 | 8,02 ± 0,32 | 3,16 ± 0,07 |
| 3 | 1 | 19,6 ± 2,6 | 9,26 ± 0,51 | 3,13 ± 0,13 |
| | 71 | 24,2 ± 1,4 | 8,04 ± 0,66 | 3,18 ± 0,08 |
| | 122 | 24,8 ± 0,9 | 6,43 ± 0,60 | 3,16 ± 0,13 |
| 6-7 | 1 | 19,2 ± 2,7 | 9,18 ± 0,26 | 3,17 ± 0,05 |
| | 71 | 25,2 ± 3,4 | 8,75 ± 0,19 | 3,64 ± 0,12 |
| | 122 | 24,9 ± 3,9 | 7,92 ± 0,28 | 3,71 ± 0,24 |
| 8 | 1 | 19,2 ± 1,6 | 8,72 ± 0,24 | 3,17 ± 0,04 |
| | 71 | 22,6 ± 3,1 | 6,25 ± 0,21 | 3,19 ± 0,08 |
| | 122 | 21,4 ± 4,3 | 5,38 ± 0,32 | 3,32 ± 0,18 |
| 9 | 1 | 17,8 ± 2,9 | 8,22 ± 0,24 | 3,16 ± 0,04 |
| | 71 | 19,1 ± 1,9 | 7,95 ± 0,19 | 3,16 ± 0,05 |
| | 122 | 23;4 ± 3,7 | 6,42 ± 0,73 | 3,17 ± 0,07 |

Gruppen 4 und 5 (mit nicht arthritogenen Proteoglycanen immunisierte Tiere):

Die Tiere der Gruppen 4 und 5 wurden mit Proteoglycanen aus bovinen Gelenkknorpeln (BAC) immunisiert, aber nur Gruppe 5 wurde mit ET-18-$OCH_3$ behandelt (Tabelle I). Wie erwartet, entwickelte keines dieser Tiere der Gruppen 4 und 5 Arthritis.

Die Tiere in Gruppe 4 zeigten eine "normale" Antikörper-Produktion gegenüber immunisierendem bovinen Gelenk-Proteoglycan und einem kontinuierlich steigenden Antikörpertiter gegenüber kreuzreagierenden, aber nicht arthritogenen Epitopen auf menschlichen (HYAC) und Maus-Proteoglycanen (Fig. 8). Obwohl Antikörper gegen Knorpel-Protoglycane der Maus gefunden wurden, wird angenommen, daß diese kreuzreagierenden Antikörper nicht an der Entwicklung der Krankheit beteiligt sind. Die als Stimulationsindex ausgedrückte Lymphozyten-Antwort (Mikecz et al., supra) wurde in Gegenwart sowohl von BAC als auch von HYAC-Proteoglycanen signifikant gesteigert (Fig. 7).

Die Tiere der Gruppe 5 (Tabelle I) erzeugten einen geringeren Antikörperspiegel sowohl gegenüber bovinen (Ergebnisse nicht gezeigt) oder menschlichen Proteoglycanen, und Auto-Antikörper gegen Knorpel-Proteoglycane der Maus wurden selbst bei einer 1:100 Serumverdünnung kaum gefunden (Fig. 9). Der Spiegel von zirkulierenden Antikörpern wurde nach einer zweiwöchigen Behandlung mit ET-18-$OCH_3$ verringert, wobei dieser Effekt bei einer Dosis von 25 mg/kg pro Tag charakteristischer als bei einer Dosis von 20 mg/kg pro Tag war. Die Antikörper-Erzeugung nahm jedoch kontinuierlich während der gesamten Behandlungsdauer zu (Fig. 9). Die Lymphozyten-Stimulation sowohl mit Proteoglycan-Antigenen oder ConA wurde deutlich supprimiert, sogar nach Beendigung der oralen Behandlung mit ET-18-$OCH_3$ (Fig. 7).

Gruppen 6 und 7 (mit HYAC immunisierte, aber nicht mit ET-18-$OCH_3$ behandelte Tiere):

Tiere dieser beiden Gruppen (Tabelle I) wurden immunisiert und wie bereits beschrieben getestet (Glant et al., Arthritis Rheum. 30 (1987), 201-212; Mikecz et al., Arthritis Rheum. 30 (1987), 306-318). Es wurde kein Unterschied zwischen Tieren der Gruppe 6 und 7 festgestellt, wobei letztere Gruppe täglich mit Milch ohne ET-18-$OCH_3$ während der gesamten Behandlungsdauer gefüttert wurde. Die Tiere erzeugten Antikörper gegen HYAC-Proteoglycane und einen hohen Spiegel an Auto-Antikörpern gegen Knorpel-Proteoglycane der Maus. Alle Tiere entwickelten Arthritis nach der dritten, oder sofort nach der vierten intraperitonealen Injektion von HYAC-Proteoglycan (Fig. 10), die progressiv war und zu Deformierungen und zur Ankylose von peripheren Gelenken wie bereits beschrieben führte (Glant et al., Arthritis rheum. 30 (1987), 201-212). Die in vitro Proliferation von Splenozyten dieser arthritischen Tiere sind in Fig. 11 zusammengefaßt, welche die charakteristische Stimulierung von Lymphozyten in Gegenwart von Proteoglycan-Antigenen und ConA zeigt.

Gruppe 8 (mit HYAC immunisierte und mit ET-18-$OCH_3$ behandelte Tiere):

6 Tiere der Gruppe 8 wurden mit Proteoglycanen von HYAC (wie die Tiere der Gruppen 6 und 7) immunisiert, sie wurden jedoch 12 Wochen lang mit ET-18-$OCH_3$ behandelt. Da sich die Tiere in einem physisch schlechten Zustand befanden und zwei Tiere in der dritten und fünften Behandlungswoche starben, mußte die ET-18-$OCH_3$-Dosis von 25 mg/kg pro Tag auf 20 mg/kg pro Tag von der sechsten Woche der Behandlung an verringert werden (Fig. 12). Diese Verringerung der Dosis half insofern, als sich die Tiere, die zuvor in schlechter physischer Verfassung waren, erholten. 3 von 4 Tieren dieser Gruppe entwickelten Arthritis, aber das Auftreten von Arthritis fand erheblich später statt und alle klinischen Symptome (z.B. Rötung und Schwellung, ausgedrückt als intermalleolarer Durchmesser) waren milder und die Zahl der befallenen Gelenke war geringer als in den Gruppen 6 und 7 (Tabelle 2).

Tiere dieser Gruppe (Nr. 8) und aller behandelter Gruppen (Nr. 3, 5 und 9) waren etwas anämischer als Tiere in anderen Gruppen (Nr. 2, 4, 6 und 7), die nicht mit ET-18-$OCH_3$ behandelt wurden (Tabelle II). Dies kann entweder die Folge einer direkten Wirkung von ET-18-$OCH_3$ auf Knochenmarkszellen oder eine indirekte Wirkung der allgemein schlechteren physischen Verfassung sein, die z.B. durch das Körpergewicht in Tabelle II ausgedrückt ist. Jedoch wurde bei allen Mäusen eine leichte Anämie als Folge der häufigen Blutabnahme beobachtet. Andere Laborparameter, wie z.B. Proteinurie (nicht festgestellt), die Zahl und das Verhältnis der weißen Zellen, zeigten keinen signifikanten Unterschied zwischen behandelten und nicht behandelten Tieren.

Die Spiegel von zirkulierenden Antikörpern gegen Proteoglycane von HYAC und Maus-Knorpeln wurde von der zweiten Behandlungswoche an supprimiert, er begann sich jedoch nach einem kurzen Zeitraum sogar während der Behandlung mit der höheren (25 mg/kg/Tag) Dosis von ET-18-$OCH_3$ zu erhöhen (Fig. 12). Andererseits wurde die Antigen-stimulierte und nicht spezifische (ConA-induzierte) T-Lymphozyten-Proliferation bei allen behandelten Tieren gleichermaßen supprimiert (Fig. 11).

Gruppe 9 vor der Immunisierung (mit arthritogenem Proteoglycan mit ET-18-$OCH_3$ vorbehandelt):

Tiere (ursprünglich 10) dieser Gruppe wurden mit ET-18-$OCH_3$ 2 Wochen vor der ersten Injektion des Antigens oral behandelt. Unglücklicherweise starben 4 Tiere dieser vorbehandelten Gruppe innerhalb der ersten 6 Wochen des Experiments, was darauf hinwies, daß die 25 mg/kg/Tag Dosis von ET-18-$OCH_3$ vermutlich eine zu hohe Dosis für Balb/c-Mäuse ist. Aus Vergleichsgründen wurde jedoch die ET-18-$OCH_3$-Dosis nicht verringert, bis zwei Tiere in Gruppe 8 starben (siehe oben).

Die klinischen Parameter und der physische Zustand der Tiere waren gleich wie in Gruppe 8, abgesehen davon, daß diese vorbehandelten Tiere keine klinischen Symptome von Arthritis zeigten. Die Lymphozyten-Stimulation mit Proteoglycanen von bovinen oder menschlichen Knorpeln sowie mit Concavalin A war gleich wie bei Tieren der Gruppe 8 (d. h. es gab eine starke Suppression sowohl der spezifischen als auch der nicht-spezifischen T-Lymphozyten-Antwort). Weiterhin wurde die Produktion von Antikörpern gegen Knorpel-Proteoglycane signifikant verzögert und die Antikörper wurden im Serum gewöhnlich erst nach der vierten Antigen-Injektion festgestellt.

Schlußfolgerung:

Die Behandlung der Balb/c-Mäuse mit ET-18-$OCH_3$ ist in der Lage, akute Entzündungsvorfälle zu supprimieren und blockiert das Voranschreiten von chronischer Arthritis bei Proteoglycan-induzierter Arthritis. Weiterhin scheint eine Vorbehandlung von Tieren mit ET-18-$OCH_3$ Balb/c-Mäuse vor dem Auftreten von Entzündungen zu schützen, wahrscheinlich aufgrund der Suppression einer T-Zellen vermittelnden Immunantwort. Die Produktion von (Auto)-Antikörpern wurde signifikant während der Behandlung supprimiert, die als Antikörper-Titer gegen menschliche und Maus Knorpel-Proteoglycane bestimmt wurden. Dieser Suppressionseffekt ist vermutlich für 3 bis 4 Wochen der Antikörperproduktion nach oraler Verabreichung von ET-18-$OCH_3$ konstant und wird dann durch einen unbekannten Mechanismus wieder aufgehoben.

**Patentansprüche**

1. Verwendung von Verbindungen der allgemeinen Formel I

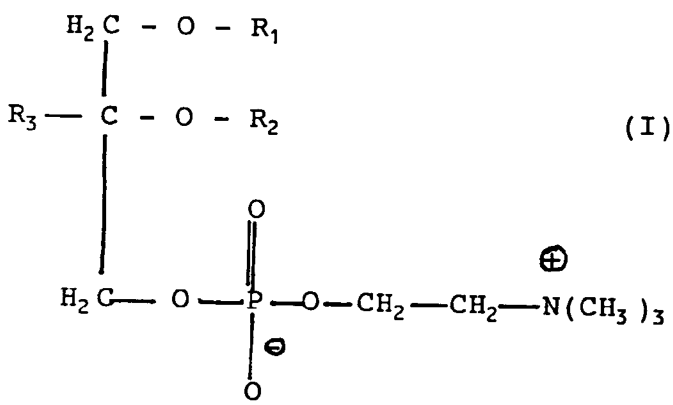

(I)

in der
$R_1$ Alkyl mit 12 bis 18 Kohlenstoffatomen,
$R_2$ Alkyl mit 1 bis 8 Kohlenstoffatomen und
$R_3$ H oder Alkyl mit 1 bis 3 Kohlenstoffatomen darstellt
zur Herstellung einer pharmazeutischen Zubereitung zur Behandlung von rheumatoider Arthritis oder ankylosierende Spondylitis.

2. Verwendung nach Anspruch 1,
**dadurch gekennzeichnet**,
daß in der allgemeinen Formel (I) $R_2$ eine Alkylgruppe mit 1 bis 3 Kohlenstoffatomen bedeutet, und $R_1$ und $R_3$ die in Anspruch 1 angegebene Bedeutung besitzen.

3. Verwendung nach einem der Ansprüche 1 oder 2,

**dadurch gekennzeichnet,**
daß eine Verbindung der allgemeinen Formel (I) der Struktur

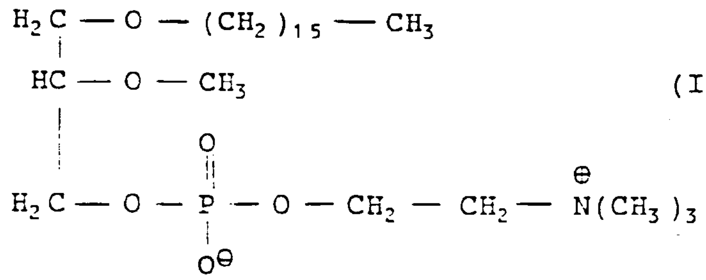

(II)

verwendet wird.

4. Verwendung nach einem der Ansprüche 1 oder 2,
**dadurch gekennzeichnet,**
daß eine Verbindung der allgemeinen Formel (I) der Struktur

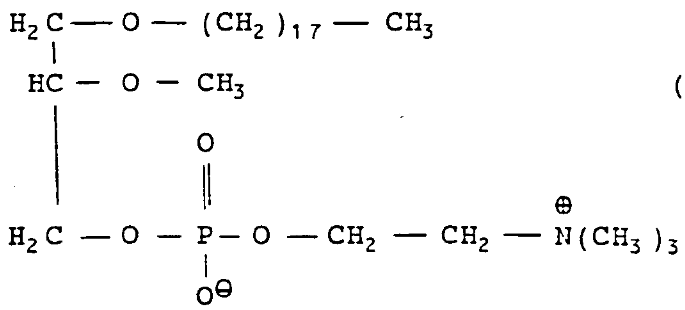

(III)

verwendet wird.

## Claims

1. Use of compounds of the general formula I

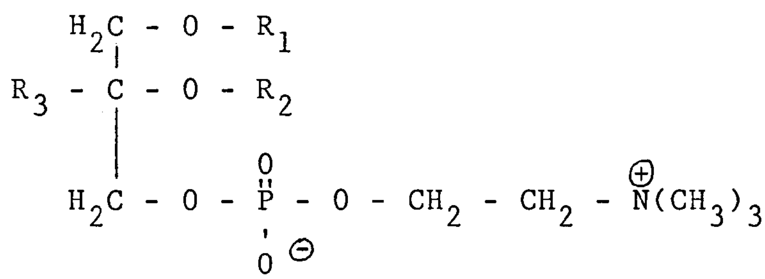

(I)

in which $R_1$ represents alkyl with 12 to 18 carbon atoms, $R_2$ alkyl with 1 to 8 carbon atoms and $R_3$ H or alkyl with 1 to 3 carbon atoms for the preparation of a pharmaceutical composition for the treatment of rheumatoid arthritis or ankylosing spondylitis.

2. Use according to claim 1, characterised in that, in general formula (I), $R_2$ signifies an alkyl group with 1 to 3 carbon atoms and $R_1$ and $R_3$ possess the meaning given in claim 1.

3. Use according to one of claims 1 or 2, characterised in that a compound of the general formula (I) of the structure:

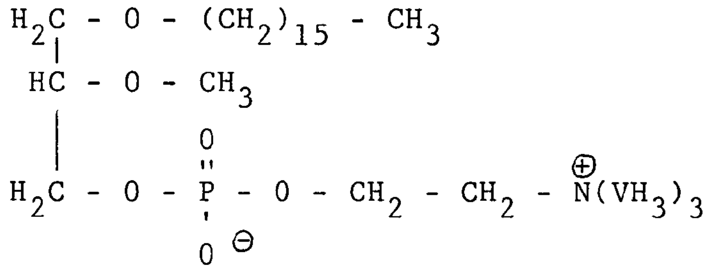

(II)

is used.

4. Use according to one of claims 1 or 2, characterised in that a compound of the general formula (I) of the structure:

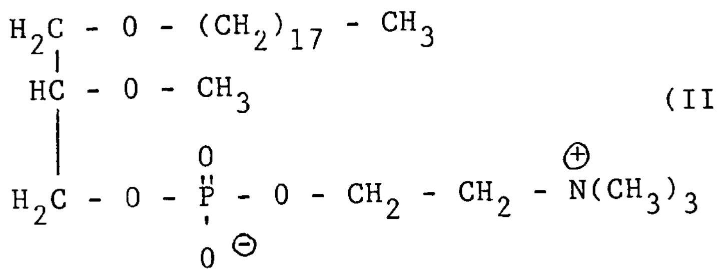

(III)

is used.

**Revendications**

1. Utilisation de composés de formule générale I

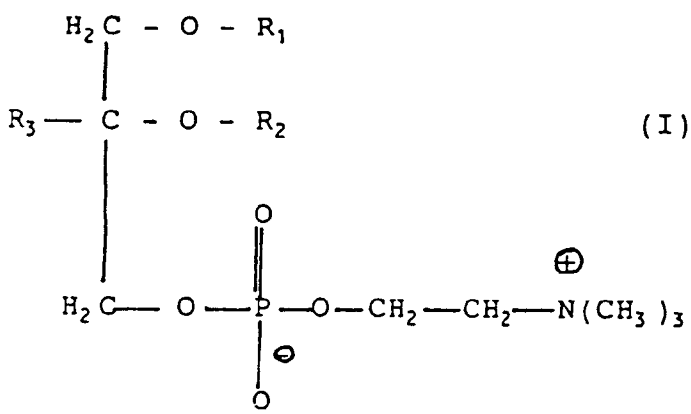

(I)

dans laquelle
$R_1$ représente un alkyle de 12 à 18 atomes de carbone
$R_2$ représente un alkyle de 1 à 8 atomes de carbone et
$R_3$ représente H ou un alkyle de 1 à 3 atomes de carbone, pour la préparation d'une composition pharmaceutique pour le traitement de la polyarthrite rhumatoïde ou de la spondylarthrite ankylosante.

2. Utilisation selon la revendication 1, caractérisée en ce que, dans la formule générale (I), $R_2$ représente un groupe alkyle de 1 à 3 atomes de carbone, et $R_1$ et $R_3$ ont la signification donnée dans la revendication 1.

3. Utilisation selon l'une des revendications 1 ou 2, caractérisée en ce que l'on utilise un composé de formule générale (I) ayant la structure

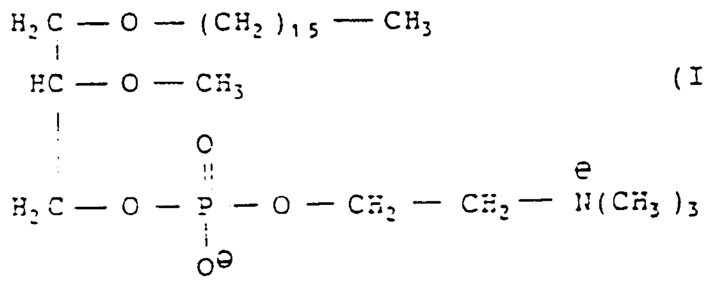

(II)

4. Utilisation selon l'une des revendications 1 ou 2, caractérisée en ce que l'on utilise un composé de formule générale (I) ayant la structure

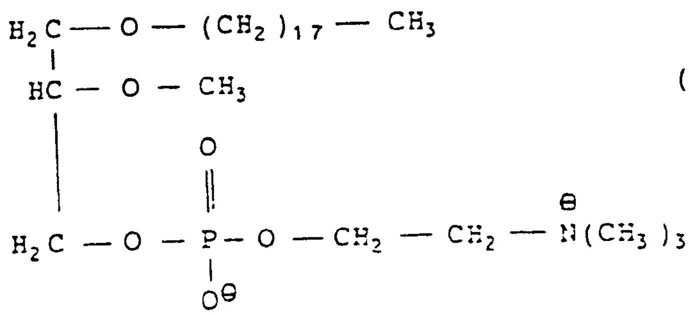

(III)

Fig.1

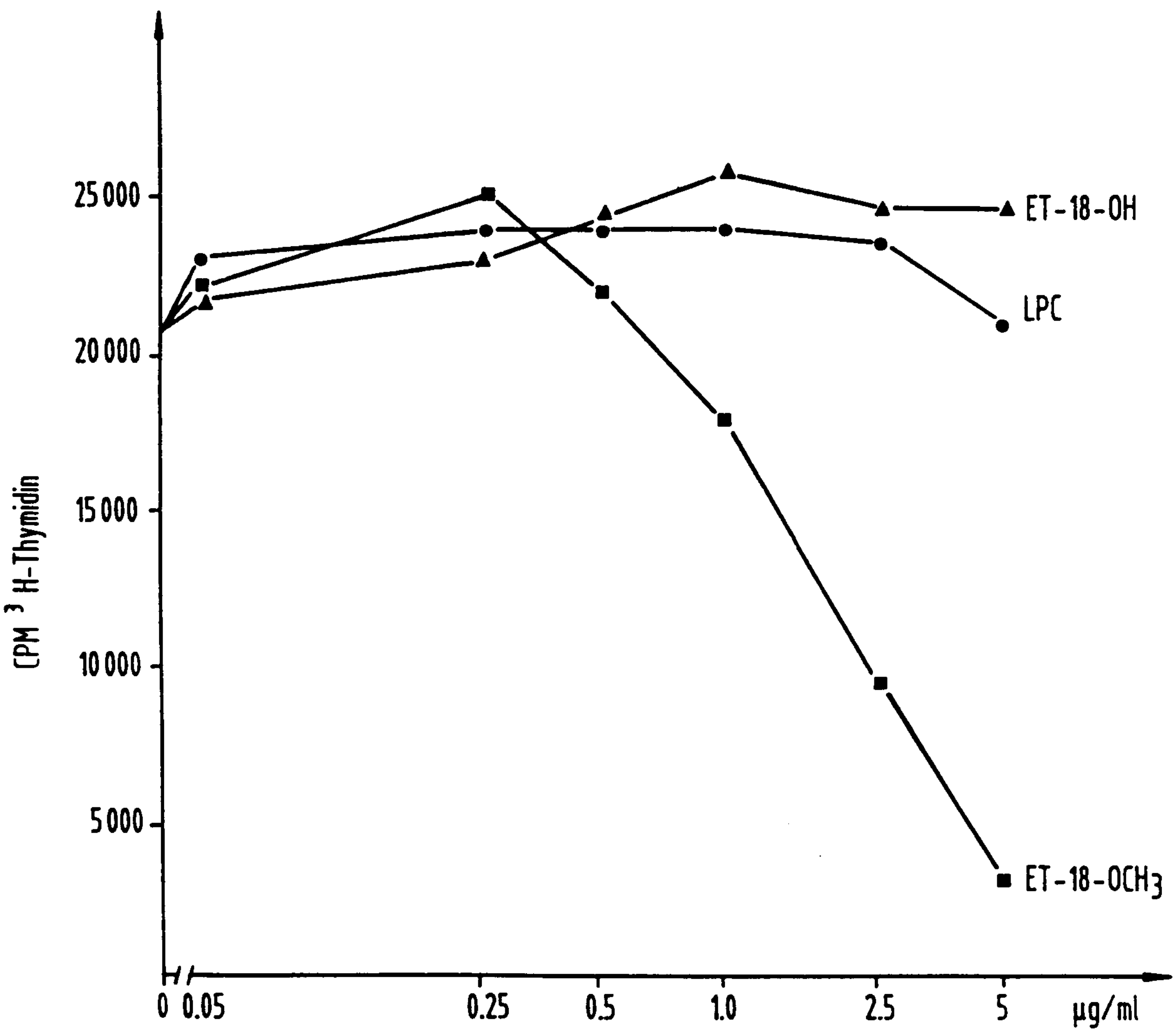
25 000
20 000
15 000
10 000
5000
CPM ³ H-Thymidin
0
0.05
0.25
0.5
1.0
2.5
5
µg/ml
ET-18-OH
LPC
ET-18-OCH3

Fig.2

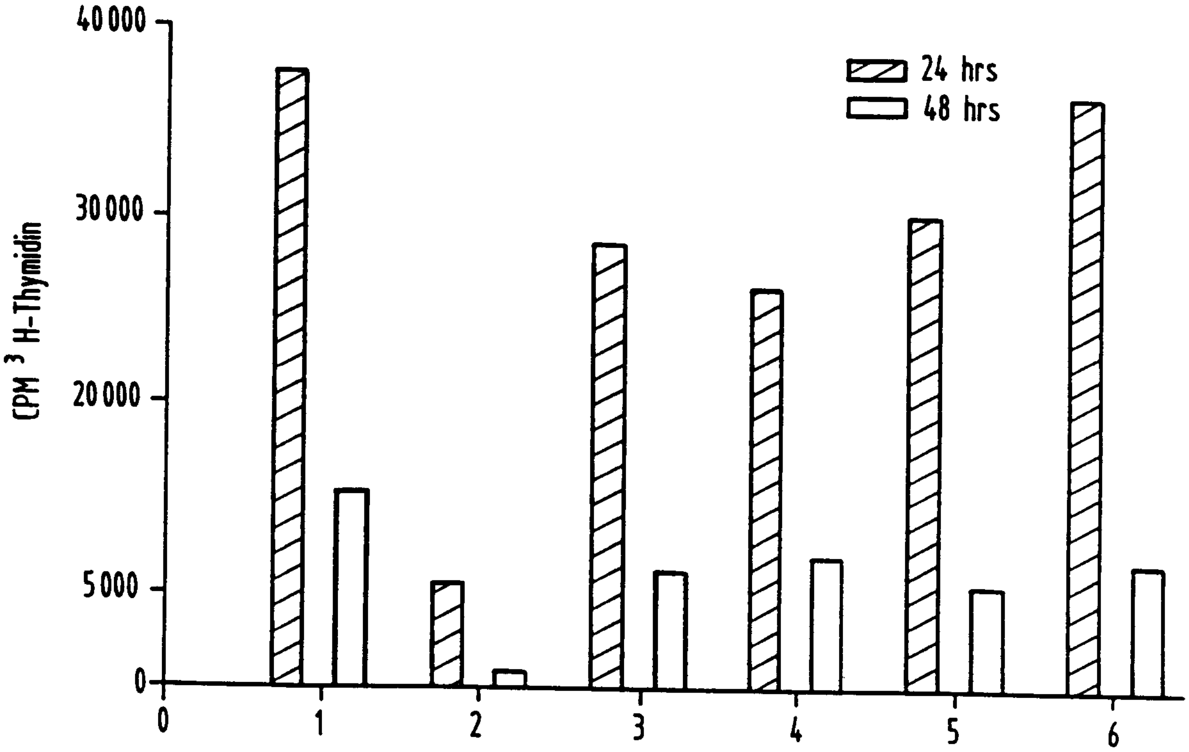


1 Kontrolle 2 ET-18-OCH3 2µg/ml 3 (D)-ET-16-OH 2µg/ml
4 (L)-ET-16-OH 2µg/ml 5 (Rac)-ET-16-OH 2µg/ml 6 LPC 2µg/ml

Fig.3

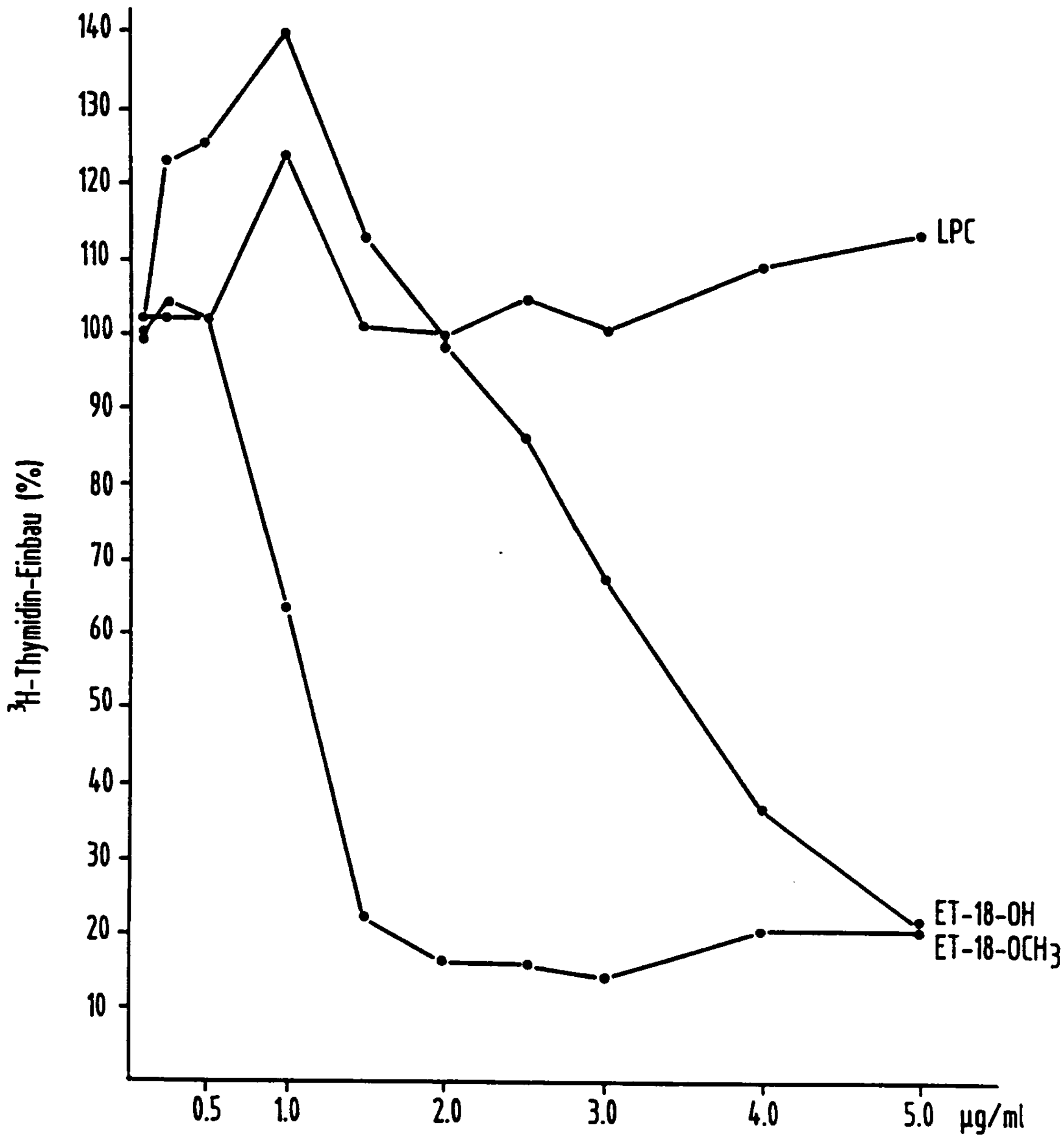
140
130
120
110
100
90
80
70
60
50
40
30
20
10
³H-Thymidin-Einbau (%)
LPC
ET-18-OH
ET-18-OCH3
0.5
1.0
2.0
3.0
4.0
5.0
µg/ml

Fig. 4

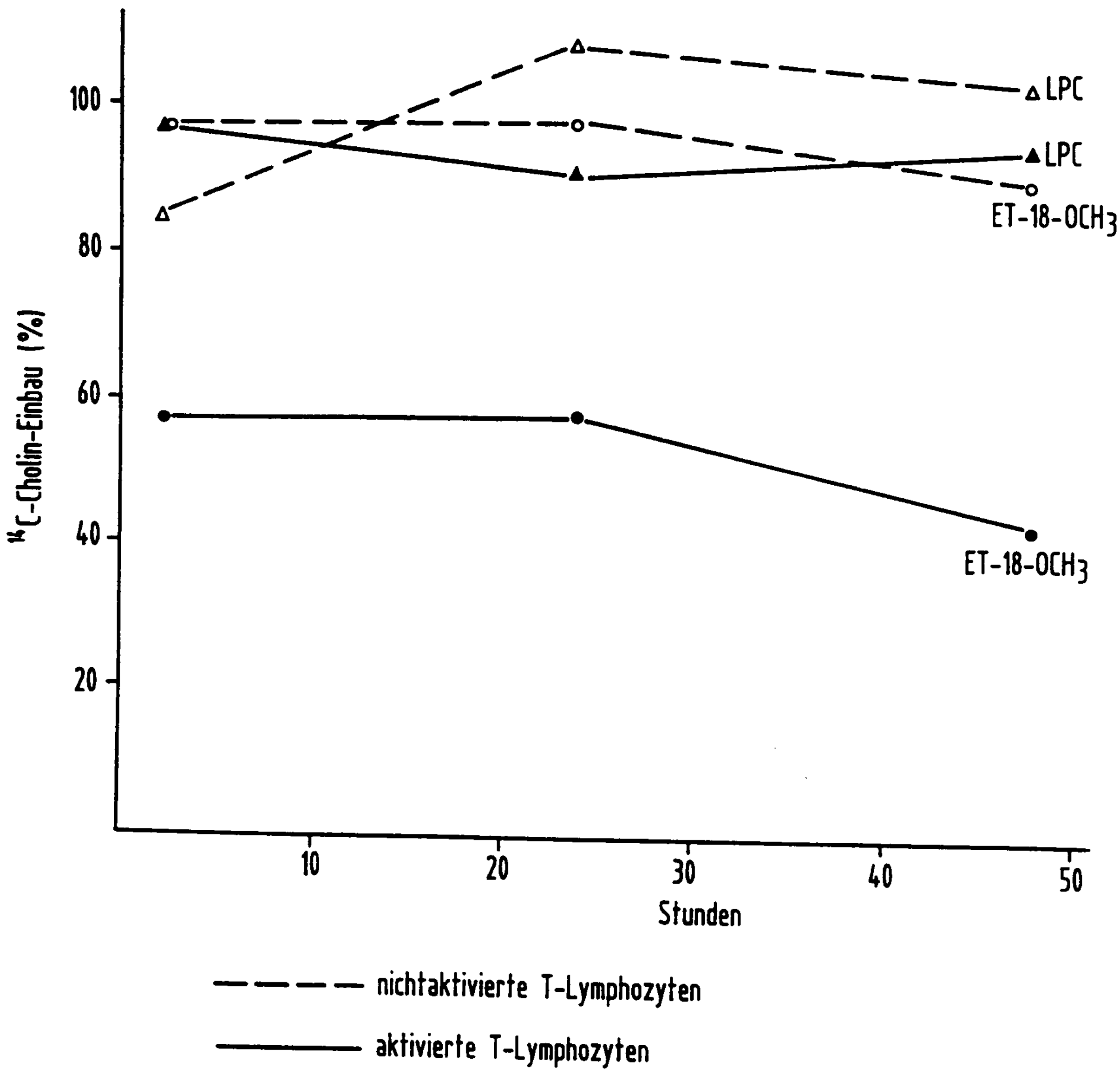

LPC
LPC
ET-18-OCH3
ET-18-OCH3
100
80
60
40
20
14C-Cholin-Einbau (%)
10
20
30
40
50
Stunden
nichtaktivierte T-Lymphozyten
aktivierte T-Lymphozyten

Fig.5

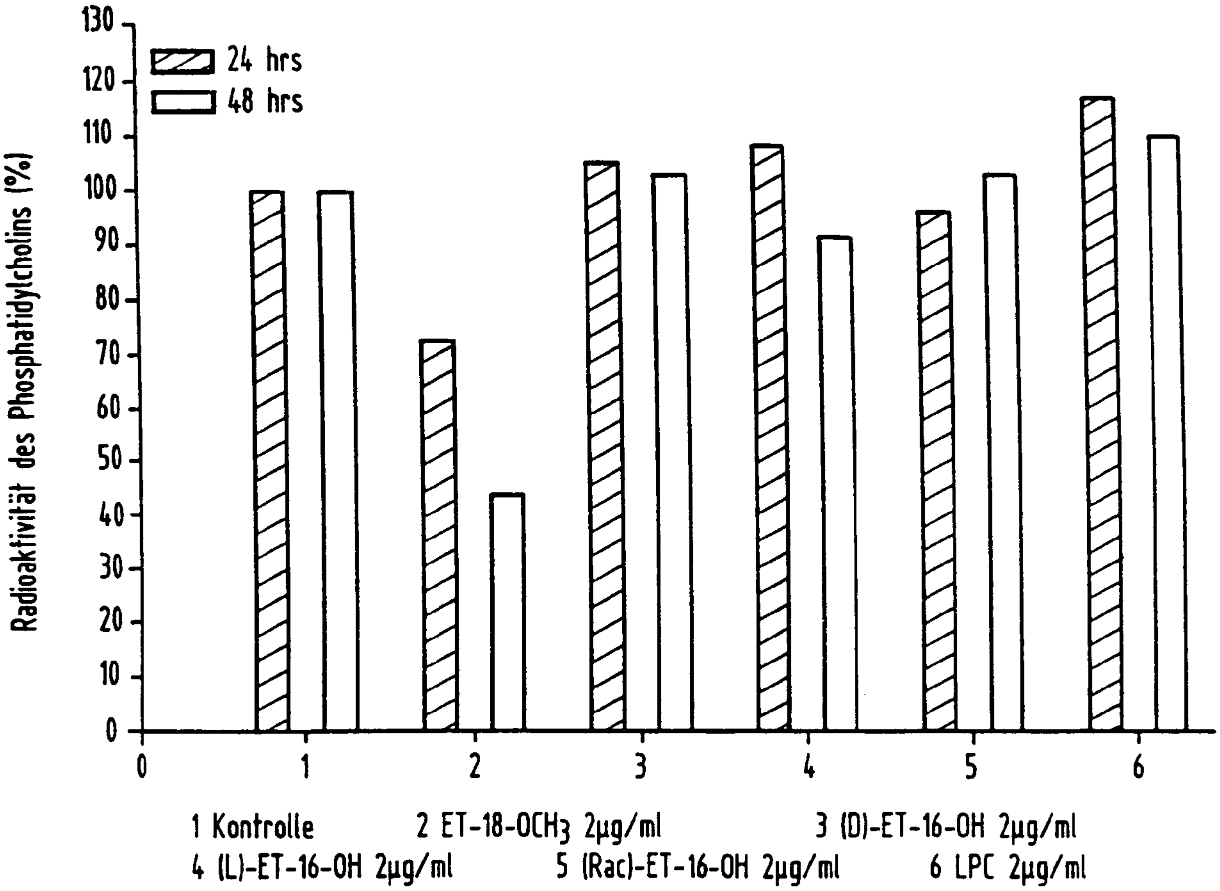
130
120
110
100
90
80
70
60
50
40
30
20
10
0
24 hrs
48 hrs
Radioaktivität des Phosphatidylcholins (%)
0
1
2
3
4
5
6
1 Kontrolle
2 ET-18-OCH3 2µg/ml
3 (D)-ET-16-OH 2µg/ml
4 (L)-ET-16-OH 2µg/ml
5 (Rac)-ET-16-OH 2µg/ml
6 LPC 2µg/ml

Fig. 6

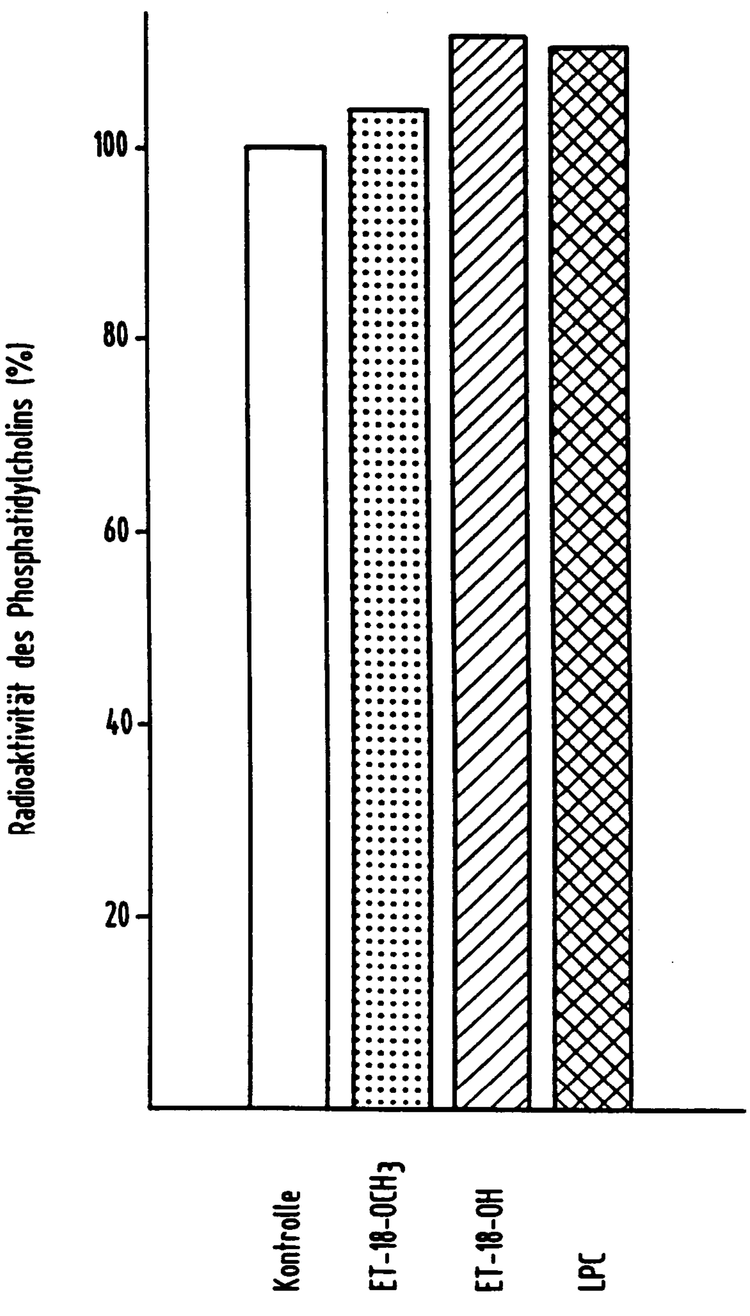
Radioaktivität des Phosphatidylcholins (%)
100
80
60
40
20
Kontrolle
ET-18-OCH3
ET-18-OH
LPC

Fig.7

Lymphozyten-Stimulationstest

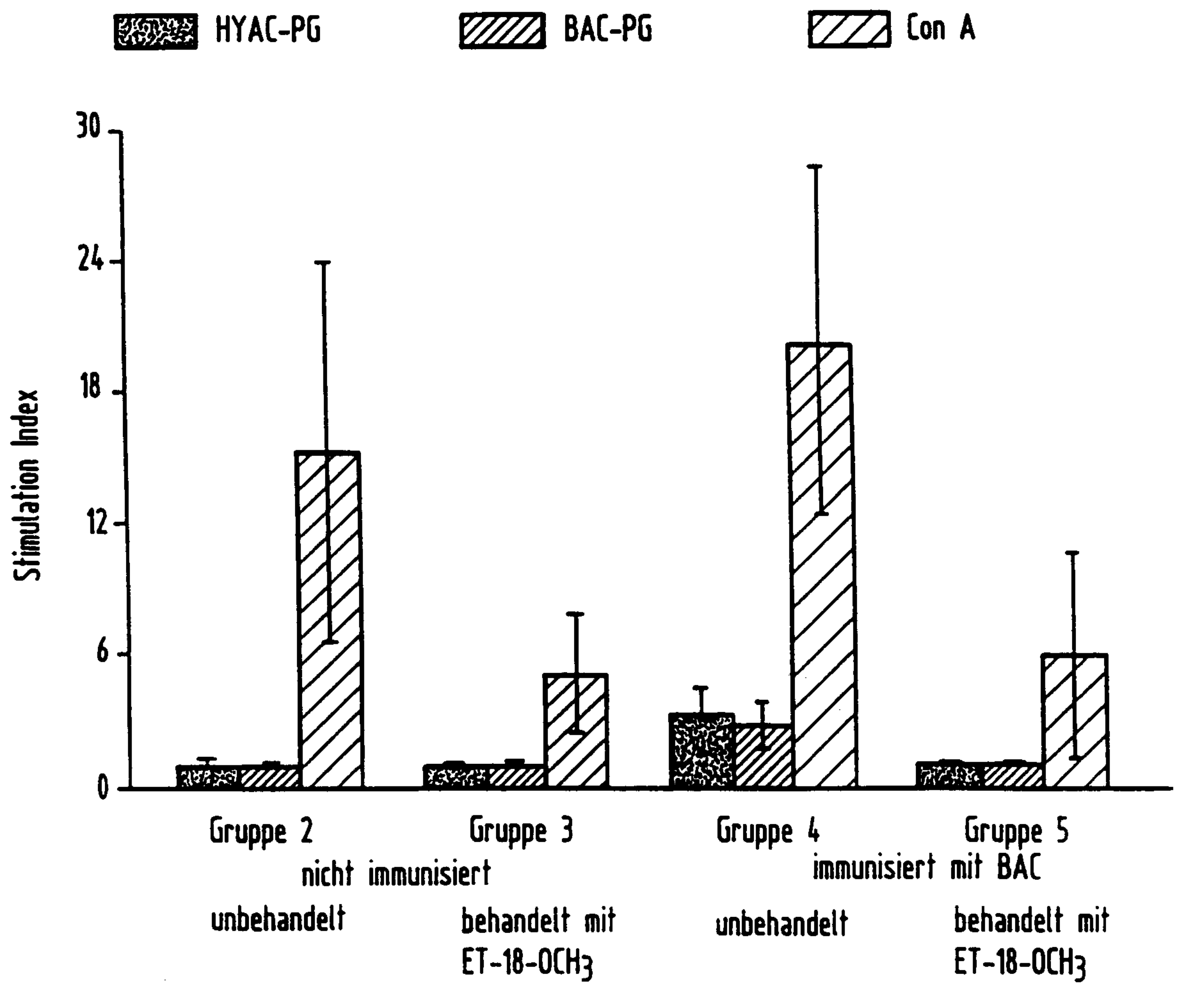

# Fig.8

Serum-Antikörperspiegel von Gruppe 4

Die Pfeile zeigen die Tage der Antigen-Injektionen

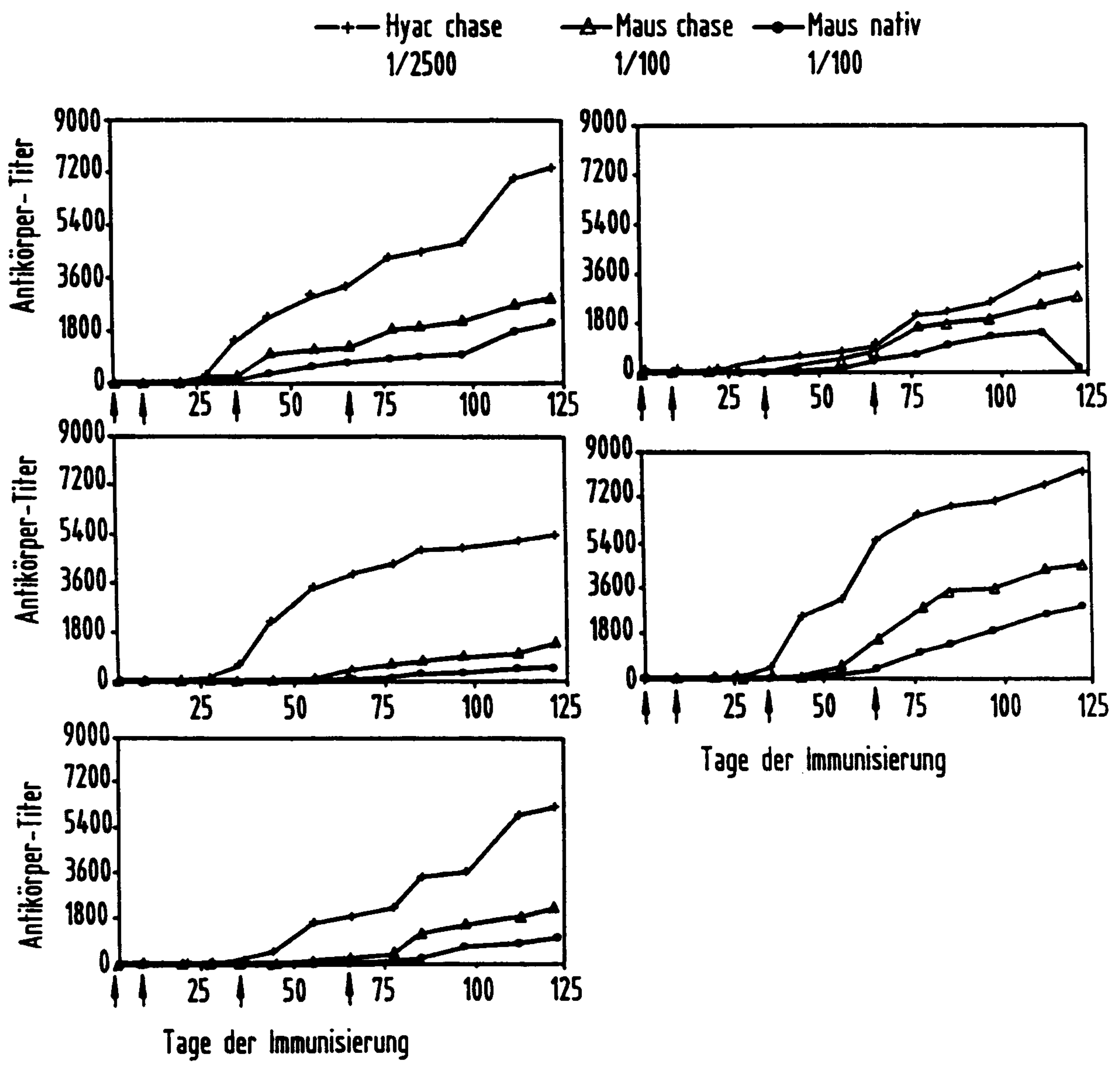

# Fig.9

Wirkung von ET-18-OCH3 auf den Serum-Antikörperspiegel von Gruppe 5

Die Behandlung mit ET-18-OCH3 wurde mit 25 mg/kg/Tag bei Woche 6 der Immunisierung begonnen und von Woche 12 an auf 20 mg/kg/Tag reduziert. Die Pfeile zeigen die Tage der Antigen-Injektionen.

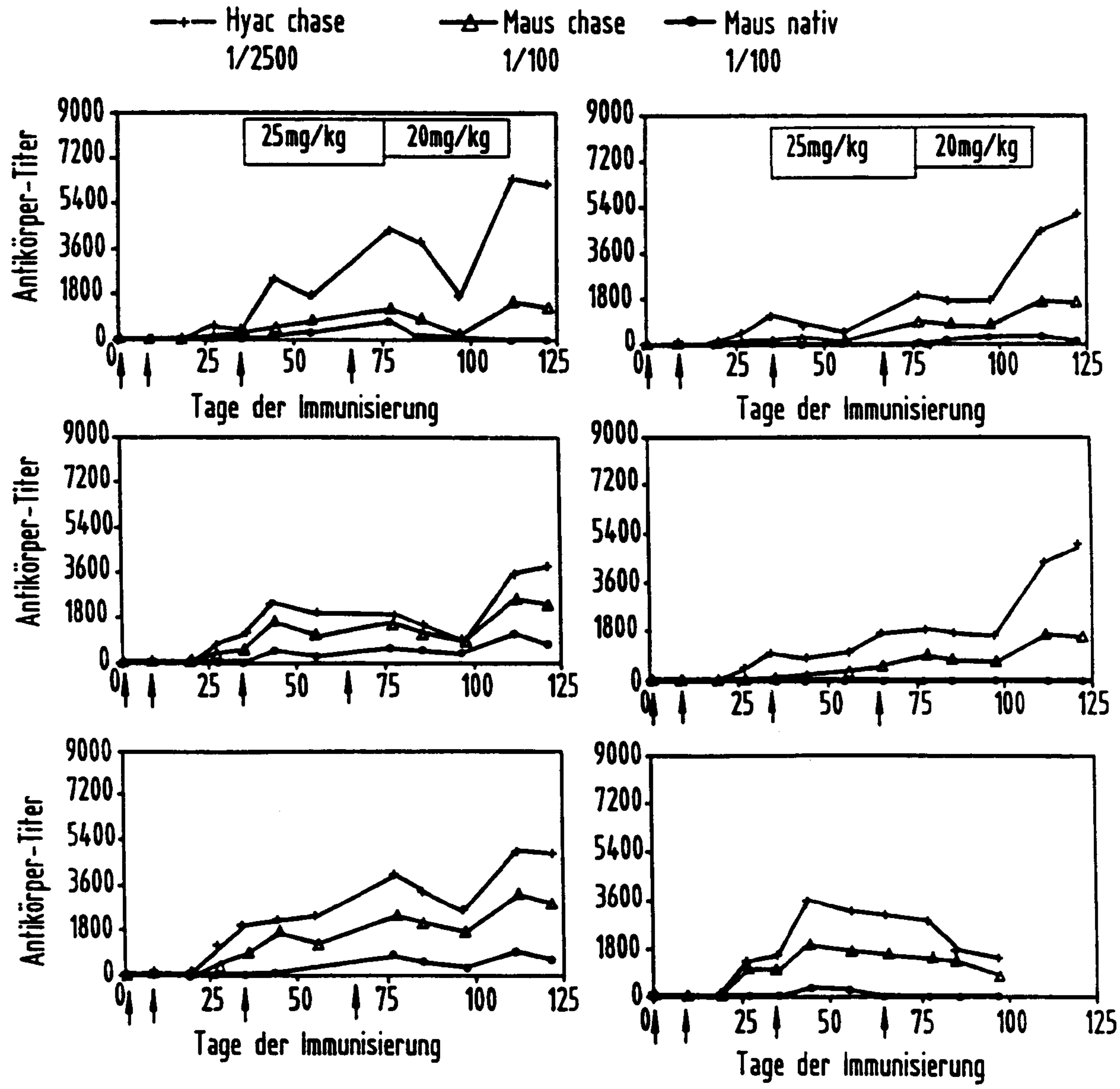

Fig.10

Serum-Antikörper-Spiegel von Gruppe 7

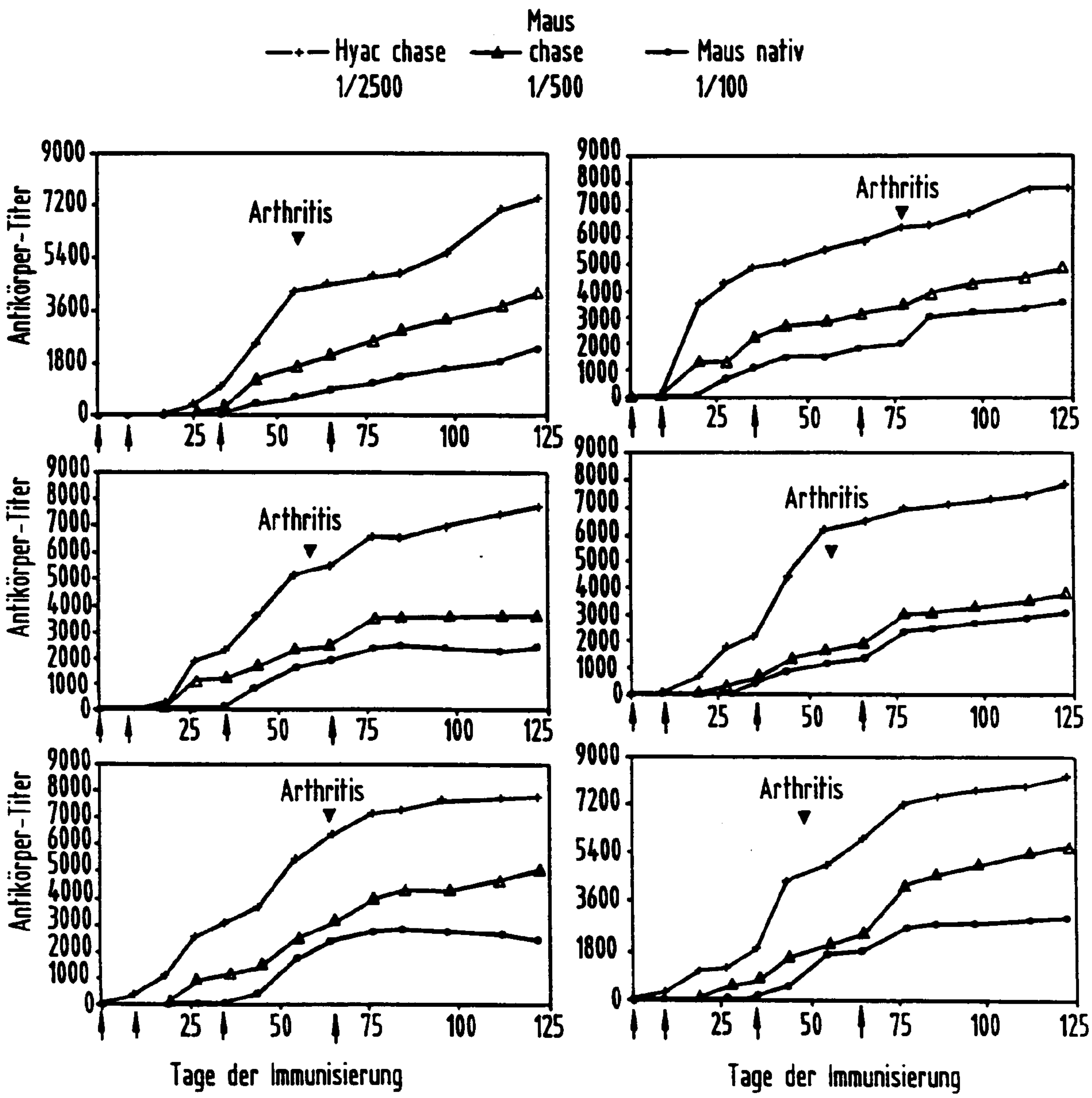

Fig.11

Lymphozyten-Stimulationstest

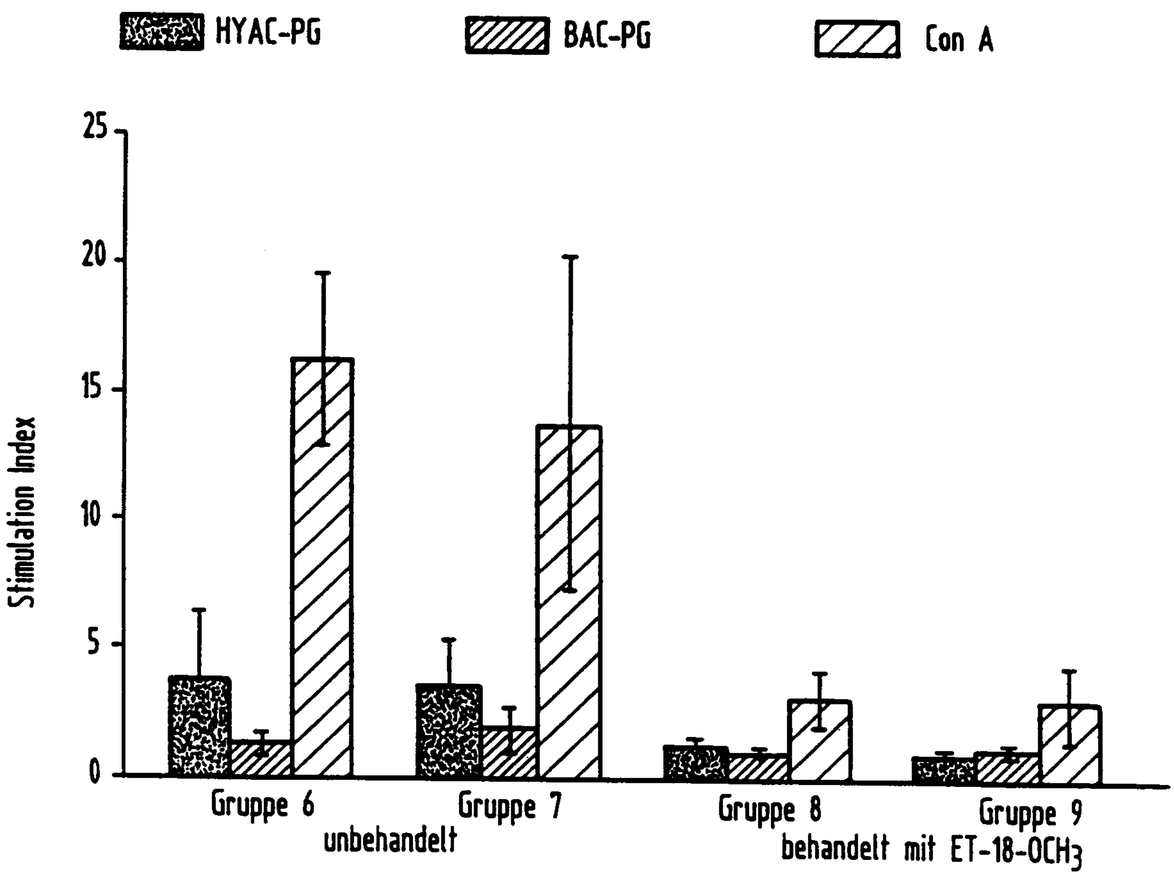

# Fig.12

Wirkung von ET-18-OCH3 auf den Serum-Antikörperspiegel von Gruppe 8

Die Behandlung mit ET-18-OCH3 wurde mit 25 mg/kg/Tag bei Woche 6 der Immunisierung begonnen und von Woche 12 an auf 20 mg/kg/Tag reduziert. die Pfeile zeigen die Tage der Antigen-Injektionen.

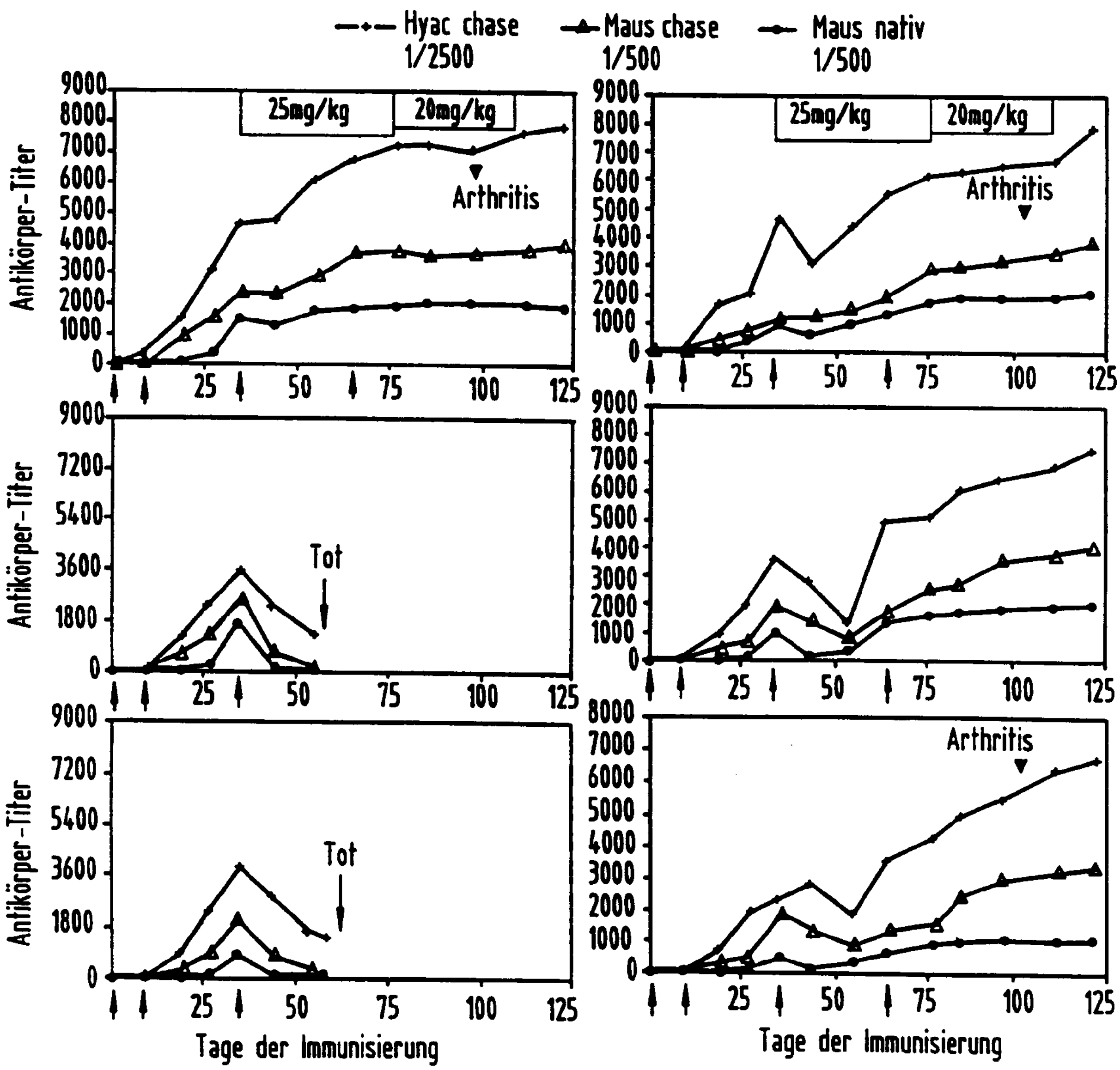

# Fig.13

Die Behandlung mit ET-18-OCH3 wurde mit 25 mg/kg/Tag 2 Wochen vor der ersten Antigen-Injektion begonnen und während der gesamten Immunisierung weitergeführt. Von der 12. Woche der Immunisierung an wird die ET-18-OCH3-Dosis auf 20 mg/kg/Tag reduziert.